# DEMANDE DE BREVET EUROPEEN

(11) **EP 1 693 026 A1**
(43) Date de publication de la demande: **23.08.2006**
(21) Numéro de dépôt: 06290274.7
(22) Date de dépôt: 17.02.2006
(51) Int. Cl.: A61F 2/38

(54) **Implant tibial et prothèse de genou unicondylienne le comportant**

(30) Priorité: 22.02.2005 FR 0501794
(71) Demandeur: Le Foll, Dominique, 95620 Parmain (FR); Benoit, Serge, 02100 Lesdins (FR)
(72) Inventeur: Le Foll, Dominique, 95620 Parmain (FR); Benoit, Serge, 02100 Lesdins (FR)
(74) Mandataire: Neyret, Daniel Jean Marie

(57) **Abrégé**

Implant tibial (1) pour prothèse de genou unicompartimentaire, comportant une plaque (2) sensiblement demi-circulaire comportant un bord rectiligne (6) et des moyens d'ancrage de l'implant (1) sur le tibia du patient, caractérisé en ce que lesdits moyens d'ancrage comportent une lame (5) s'étendant vers le bas à partir dudit bord rectiligne (6) en direction de l'extérieur de l'implant (1) selon un angle (a) compris entre 30 et 60°.

Prothèse de genou unicompartimentaire, caractérisée en ce que son implant tibial est du type précédent.

## Description

L'invention concerne le domaine des prothèses de genou, et plus précisément les implants tibiaux de prothèses unicompartimentaires.

Les implants tibiaux de prothèses de genou destinées à remplacer des éléments articulaires peuvent être du type bicompartimentaire ou unicompartimentaire. Dans le premier cas, ils se substituent à la totalité du plateau tibial, et coopèrent avec le fémur du patient ou avec un implant fémoral qu'on a substitué à la totalité de l'extrémité du fémur, un patin en polyéthylène assurant le contact entre les implants. Les prothèses unicompartimentaires ne recouvrent qu'une moitié du plateau tibial, et peuvent être utilisées lorsque seul l'un des côtés de l'articulation est lésé. Elles permettent de conserver le compartiment naturel sain de l'articulation, et de réduire le temps de récupération post-opératoire par rapport à la pose d'une prothèse bicompartimentaire.

Ces implants tibiaux de prothèses unicompartimentaires se présentent généralement sous la forme de plaques globalement demi-circulaires, comportant un ou des éléments d'ancrage pour leur fixation au tibia. Ces éléments d'ancrage comportent habituellement un aileron situé sur la face inférieure de l'implant. Cet aileron est perpendiculaire à ladite face inférieure, et est généralement médian de manière à être situé au contact du massif des épines.

Cette orientation a deux inconvénients importants. Lors de sa mise en place, elle fragilise une zone importante de l'extrémité supérieure du tibia. Elle est donc parfois à l'origine d'une fracture verticale spino-tubérosaire.

D'autre part, elle ne peut empêcher un éventuel relèvement antérieur du plateau lors d'une flexion complète du genou.

Le but de l'invention est de proposer un implant tibial de prothèse unicompartimentaire du genou ne présentant pas ces inconvénients.

A cet effet, l'invention a pour objet un implant tibial pour prothèse de genou unicompartimentaire, comportant une plaque sensiblement demi-circulaire comportant un bord rectiligne et des moyens d'ancrage de l'implant sur le tibia du patient, caractérisé en ce que lesdits moyens d'ancrage comportent une lame s'étendant vers le bas à partir dudit bord rectiligne en direction de l'extérieur de l'implant en formant avec le prolongement de ladite plaque un angle (α) compris entre 30 et 60°.

L'angle (α) est de préférence égal à 45°.

La lame peut être en portion de cercle.

La lame peut être en portion d'ellipse.

La lame peut être polygonale.

L'implant peut présenter une épaisseur dégressive à partir dudit bord rectiligne.

La lame peut comporter au moins une fenêtre.

La face inférieure de la plaque peut comporter des pointes obliques.

Les pointes peuvent être parallèles à la lame.

L'implant tibial peut être du type précédent.

Comme on l'aura compris, l'invention consiste à prévoir, pour l'ancrage de l'implant tibial, une lame connectée au bord médial rectiligne de l'implant, et s'étendant obliquement vers le bas à partir dudit bord, en direction de l'extérieur de l'implant.

L'invention sera mieux comprise à la lecture de la description qui suit, donnée en référence aux figures annexées suivantes :

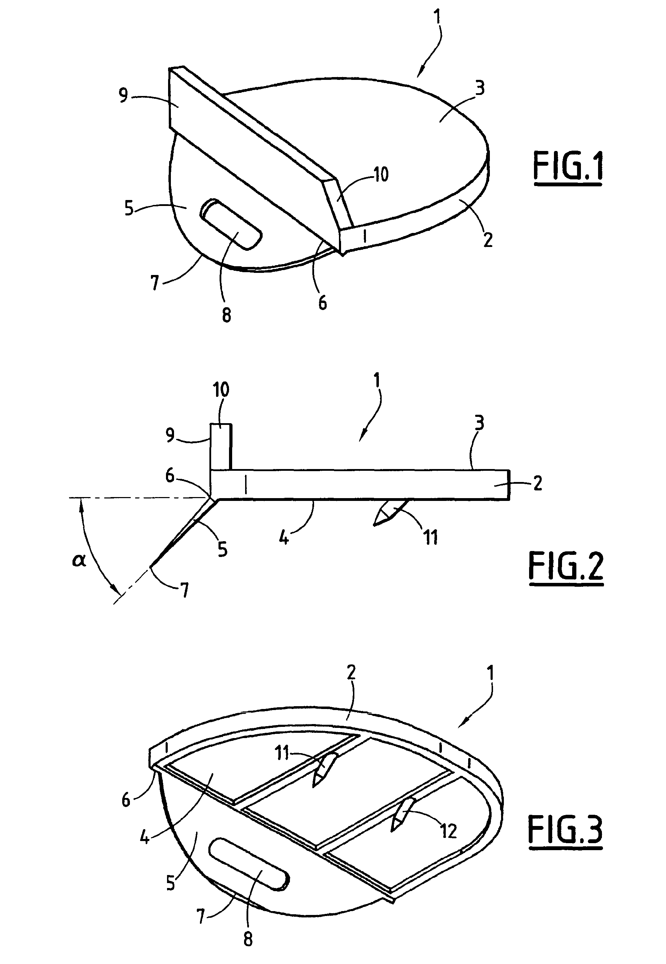

- la figure 1 qui montre, vu de dessus en perspective, un exemple d'implant tibial selon l'invention ;
- la figure 2 qui montre ce même implant tibial vu de face ;
- la figure 3 qui montre ce même implant tibial vu de dessous en perspective.

L'implant tibial 1 a, comme il est connu, une forme générale sensiblement semi-circulaire. Il comporte une plaque 2, généralement métallique, dont la face supérieure 3 est destinée à venir au contact d'un plateau mobile ou fixe en polyéthylène (non représenté), qui assurera le contact et le glissement avec le condyle fémoral du patient ou un implant fémoral qui lui aura été substitué. La face inférieure 4 de la plaque 2 est destinée à reposer sur la tête tibiale du patient après l'installation de l'implant.

Selon l'invention, l'ancrage de l'implant 1 sur le tibia du patient est assuré, au moins essentiellement, par une lame 5 en portion de cercle (dans l'exemple représenté) qui s'étend vers le bas à partir du bord rectiligne 6 de la plaque 2 en direction de l'extérieur de l'implant 1. Cette lame 5 forme un angle α avec la direction horizontale prolongeant la plaque 2, qui est de l'ordre de 30 à 60°, préférentiellement 45°.

Cette lame 5 s'insère à force par impaction dans une cavité correspondante ébauchée dans le tibia préalablement à la pose de l'implant, et qui s'étend sous le massif des épines tibiales. Elle a de préférence une épaisseur dégressive à partir de son bord de contact avec la plaque 2 : à ce niveau l'épaisseur de la lame 5 est de 2 à 3mm, alors qu'à sa périphérie 7 la lame 5 est mince et tranchante. On améliore ainsi la pénétration et l'ancrage de la lame 5 dans le tibia lors de l'impaction. Dans l'exemple représenté, la lame 5 comporte une fenêtre 8 qui, lors de la repousse osseuse suivant la pose de l'implant 1, est traversée par de la matière osseuse. Cela contribue également à l'amélioration de l'ancrage, mais cette caractéristique n'est pas obligatoire. La forme de la fenêtre peut être différente de celle représentée, et il peut y en avoir plusieurs.

La lame 5 pourrait avoir une forme différente de celle représentée, par exemple être en portion d'ellipse ou polygonale (rectangulaire ou autre).

Sur sa face supérieure 3, l'implant 1 comporte une paroi 9 sensiblement verticale s'étendant à partir de son bord rectiligne 6. Une telle paroi 9 est classique et permet d'éviter le contact entre le plateau en polyéthylène et le massif des épines. Elle peut, comme représenté, présenter un biseautage 10 sur au moins un de ses angles supérieurs pour correspondre au mieux à l'anatomie osseuse.

Sur sa face inférieure 4, l'implant 1 peut comporter des pointes 11, 12 obliques. Préférentiellement, elles sont inclinées par rapport à la plaque 2 selon un angle égal à α, et ont donc une orientation parallèle à celle de la lame 5.

Leur principale fonction est de stabiliser l'implant 1 avant son impaction finale qui assure la pénétration complète de la lame 5 dans le tibia. Elles peuvent également jouer un certain rôle dans le maintien en place de l'implant 1 après sa pose.

Il est envisageable de prévoir des moyens de fixation supplémentaires de l'implant 1 sur le tibia du patient, tels qu'un aileron vertical classique, ou un ou des tétons s'étendant à partir de la face inférieure 4, ou des vis...

La fixation de l'implant 1 peut s'effectuer avec ou sans ciment. Lorsque la fixation s'effectue sans ciment, on peut recouvrir la face inférieure 4 et la lame 5 par de l'hydroxyapatite ou tout autre matériau stimulant la croissance osseuse.

Les dimensions de l'implant 1 doivent, bien entendu, être choisies en fonction de l'anatomie du patient.

A titre d'exemple non limitatif, les principales dimensions d'un exemple d'implant 1 selon l'invention peuvent être, si l'implant est classiquement réalisé en un alliage Cr-Co, ou en titane nitruré :
- longueur antéro-postérieure de la plaque 2 : 47mm ;
- largeur de la plaque 2 : 29,5mm ;
- épaisseur de la plaque 2 : 2 à 3mm ;
- hauteur de la paroi 9 : 6 à 8mm ;
- longueur de la lame 5 : 10mm ;
- épaisseur de la lame 5 : de 2 à 3mm au niveau de son contact avec la plaque 2, 0,5 à 1 mm à sa périphérie;
- longueur des pointes 11, 12 : 1,5 à 2mm.

Comme on l'a dit, l'implant 1 selon l'invention permet d'éviter une fracture qui pourrait provoquer une séparation du plateau tibial. Par ailleurs, l'orientation donnée à la lame 5 permet de lutter efficacement contre les tendances au soulèvement de l'implant 1, grâce à une neutralisation des forces d'arrachement vertical.

De plus, en cas d'ablation de l'implant 1, on limite le sacrifice osseux.

Par rapport aux ailerons médians classiques, l'implantation de la lame 5 s'effectue dans une zone plus solide, et surtout plus favorable à la repousse osseuse pour les modèles sans ciment, en raison de la qualité de son os spongieux et non scléreux.

Cette configuration permet également de lutter contre une éventuelle bascule en varus.

Enfin, elle augmente la surface d'appui par rapport aux configurations classiques, et permet donc de mieux répartir les contraintes en pression sur l'extrémité supérieure du tibia. Celles-ci peuvent être parfois trop intenses avec les configurations classiques, et être alors sources de douleurs pour le patient.

L'implant tibial selon l'invention peut être intégré à une prothèse de genou unicompartimentaire dont les autres composants (notamment la partie fémorale) peuvent être de configuration tout à fait habituelle.

## Revendications

1. Implant tibial (1) pour prothèse de genou unicompartimentaire, comportant une plaque (2) sensiblement demi-circulaire comportant un bord rectiligne (6) et des moyens d'ancrage de l'implant (1) sur le tibia du patient, **caractérisé en ce que** lesdits moyens d'ancrage comportent une lame (5) s'étendant vers le bas à partir dudit bord rectiligne (6) en direction de l'extérieur de l'implant (1) en formant avec le prolongement de ladite plaque (2) un angle (α) compris entre 30 et 60°.

2. Implant selon la revendication 1, **caractérisé en ce que** l'angle (α) est égal à 45°.

3. Implant selon la revendication 1 ou 2, **caractérisé en ce que** la lame (5) est en portion de cercle.

4. Implant selon la revendication 1 ou 2, **caractérisé en ce que** la lame (5) est en portion d'ellipse.

5. Implant selon la revendication 1 ou 2, **caractérisé en ce que** la lame (5) est polygonale.

6. Implant selon l'une des revendications 1 à 5, **caractérisé en ce qu'**il présente une épaisseur dégressive à partir dudit bord rectiligne (6).

7. Implant selon l'une des revendications 1 à 6, **caractérisé en ce que** la lame (5) comporte au moins une fenêtre (8).

8. Implant selon l'une des revendications 1 à 7, **caractérisé en ce que** la face inférieure (4) de la plaque (2) comporte des pointes (11, 12) obliques.

9. Implant selon la revendication 8, **caractérisé en ce que** les pointes (11, 12) sont parallèles à la lame (5).

10. Prothèse de genou unicompartimentaire, **caractérisée en ce que** son implant tibial est du type selon l'une des revendications 1 à 9.
